# EUROPÄISCHE PATENTSCHRIFT

(11) **EP 3 154 606 B1**
(45) Veröffentlichungstag und Bekanntmachung des Hinweises auf die Patenterteilung: **14.10.2020**
(21) Anmeldenummer: 15728869.7
(22) Anmeldetag: 15.06.2015
(51) Int. Cl.: A61M 1/34

(54) **VORRICHTUNGEN ZUM BEREITSTELLEN EINER LÖSUNG FÜR DIE BLUTBEHANDLUNG**
DEVICES FOR PROVIDING A SOLUTION FOR THE TREATMENT OF BLOOD
DISPOSITIFS DE PRÉPARATION D'UNE SOLUTION POUR LE TRAITEMENT DU SANG

(30) Priorität: 16.06.2014 DE 102014108444
(43) Veröffentlichungstag der Anmeldung: 19.04.2017
(73) Patentinhaber: Fresenius Medical Care Deutschland GmbH, 61352 Bad Homburg (DE)
(72) Erfinder: KLEWINGHAUS, Jürgen, 61440 Oberursel (DE)
(74) Vertreter: Bobbert & Partner Patentanwälte PartmbB
(86) Internationale Anmeldenummer: PCT/EP2015/063334
(87) Internationale Veröffentlichungsnummer: WO 2015/193237

(56) Entgegenhaltungen:
- WO-A1-2010/040819
- DE-A1- 4 003 452
- DE-T2- 60 031 966
- US-A- 5 618 441
- US-B1- 6 635 026

## Beschreibung

Die vorliegende Erfindung betrifft eine Blutbehandlungsvorrichtung gemäß Anspruch 1.

Bei manchen extrakorporalen Blutbehandlungssitzungen wird einem dabei verwendeten Blutkreislauf in der Praxis ein Wirkstoff (im Folgenden kurz für Medikament, Arznei und/oder pharmakologisch wirksame Substanz) zugegeben.

Die Zugabe erfolgt dabei in der Praxis unter Verwendung eines Behältnisses, beispielsweise eines Lösungsbeutels (oder kurz: Beutels), in welchem der Wirkstoff in einer Lösung vorliegt. Die Lösung kann unterschiedlich hoch konzentriert sein; das bis zum Erreichen einer vorbestimmten Konzentration des Wirkstoffs im Blut zugegebene Volumen hängt offensichtlich von der Konzentration ab, in welcher der Wirkstoff in der Lösung vorliegt. Dabei haben sowohl hoch-konzentrierte als auch niedrig-konzentrierte Lösungen jeweils ihre Vor- und Nachteile.

Ein Vorteil von Lösungsbeuteln mit Wirkstoffen in hohen Konzentrationen ist, dass ein verwendeter Beutel vergleichsweise lange hält und Beutelwechselintervalle groß sein können, was Zeit zum Wechseln des Beutels einspart und nur wenig Lagerplatz für derartige Beutel erfordert. Ein Nachteil der Verwendung von Beuteln mit hoher Konzentration ist, dass hochkonzentrierte Lösungen eher zu ungleichmäßiger Mischung mit dem Patientenblut, wie beispielsweise Koagelbildung, neigen.

Ein Vorteil der Verwendung von Beuteln mit niedriger Konzentration ist, dass ihre Lösung eher nicht zur Koagelbildung und zugleich zu gleichmäßiger Mischung mit dem Patientenblut neigt. Ein Nachteil von Beuteln niedriger Konzentration ist, dass ein verwendeter Lösungsbeutel vergleichsweise kurz hält und Beutelwechselintervalle somit kurz sind, was Zeit zum Wechseln des Beutels kostet. Ferner erfordern solche Beutel einen vergleichsweise großen Lagerplatz und bedeuten erhöhten Aufwand bei Beschaffung und Entsorgung.

Das Dokument DE60031966 T2 offenbart einen extrakorporalen Kreislauf, wobei ein Antikoagulans und Ersatzfluids mit einer Pumpe in eine Blutleitung geleitet werden.

Eine Aufgabe der vorliegenden Offenbarung ist es, ein weiteres Verfahren und Vorrichtungen zum Bereitstellen einer Lösung für die Blutbehandlung anzugeben.

Ferner sollen eine Blutbehandlungsvorrichtung, mit welcher das offenbarte, nicht-erfindungsgemäße Verfahren durchführbar ist sowie eine zur Durchführung des Verfahrens vorgesehene Steuereinrichtung, ein geeignetes digitales Speichermedium, ein geeignetes Computerprogramm-Produkt und ein geeignetes Computerprogramm angegeben werden.

Die erfindungsgemäße Aufgabe wird durch eine Blutbehandlungsvorrichtung mit den Merkmalen des Anspruchs 1 gelöst.

Alle mit dem offenbarten, nicht-erfindungsgemäßen Verfahren erzielbaren Vorteile lassen sich auch mit den erfindungsgemäßen Vorrichtungen erzielen.

Das offenbarte, nicht-erfindungsgemäße Verfahren betrifft das Bereitstellen einer Lösung für eine Blutbehandlung eines Patienten, welche mittels einer Blutbehandlungsvorrichtung und unter Verwendung eines extrakorporalen Blutkreislaufs und/oder einer Blutkassette durchgeführt wird. Das Verfahren umfasst wenigstens das Bereitstellen einer Blutbehandlungsvorrichtung, welche zum Zweck der Blutbehandlung des Patienten die im Folgenden genannten Einrichtungen und Vorrichtungen aufweist oder mit ihnen in Fluid- und/oder Signalverbindung verbindbar oder verbunden ist.

So weist die Blutbehandlungsvorrichtung wenigstens eine erste Quelle mit einer oder für eine (vorzugsweise physiologische) Kochsalzlösung (beispielsweise eine 0,9%-NaCl-Lösung), eine Dialysierflüssigkeit oder ein Substituat (also eine Substituatflüssigkeit) auf oder ist hiermit verbunden. Sie weist ferner wenigstens eine erste Leitung auf oder ist mit dieser verbunden, welche sich stromabwärts an die erste Quelle anschließt. Das erste Fluid ist im Folgenden der Einfachheit halber als Dialysierflüssigkeit oder Substituat bezeichnet. Das erste Fluid kann, wie oben ausgeführt, beispielsweise auch eine Kochsalzlösung sein. Was im Folgenden zu Dialysierflüssigkeit oder Substituat ausgeführt ist, gilt ungemindert auch für Kochsalzlösung.

Die Blutbehandlungsvorrichtung weist ferner wenigstens eine zweite Quelle für einen Wirkstoff auf, welcher in der zweiten Quelle in Form eines Konzentrats oder einer Lösung als ein zweites Fluid vorliegt, oder ist hiermit verbunden.

Die Blutbehandlungsvorrichtung weist ferner wenigstens eine zweite Leitung auf, welche sich stromabwärts an die zweite Quelle anschließt.

Die Blutbehandlungsvorrichtung weist ferner wenigstens eine sogenannte gemeinsame Leitung auf. Diese gemeinsame Leitung steht mit einem, ggf. weiteren, Abschnitt des extrakorporalen Blutkreislaufs und/oder einem, ggf. weiteren, Abschnitt der Blutkassette, vorzugsweise mit einem arteriellen oder einem venösen Leitungsabschnitt, in Fluidverbindung. Sie wird als gemeinsame Leitung bezeichnet, weil in ihr sowohl erstes Fluid als auch zweites Fluid gemeinsam, also zur selben Zeit, strömen können. Hierzu münden sowohl die erste Leitung als auch die zweite Leitung derart in die gemeinsame Leitung oder gehen in diese über, dass sowohl die erste Quelle als auch die zweite Quelle mit der gemeinsamen Leitung in Fluidverbindung stehen und/oder in ihr das erste Fluid aus der ersten Quelle und das zweite Fluid aus der zweiten Quelle aufgrund der Anordnung der gemeinsamen Leitung in dieser zu einem kombinierten Fluid zusammentreffen können.

Die Blutbehandlungsvorrichtung weist ferner wenigstens eine erste Fördereinrichtung zum Fördern eines Fluids auf. Die erste Fördereinrichtung ist angeordnet, um ein innerhalb der gemeinsamen Leitung vorliegendes Fluid, vorzugsweise in oder innerhalb von extrakorporalem Blutkreislauf und/oder Blutkassette, zu fördern.

Das offenbarte, nicht-erfindungsgemäße Verfahren umfasst weiter das Betreiben wenigstens der ersten Fördereinrichtung derart, dass eine Kombination aus erstem Fluid und zweitem Fluid durch die gemeinsame Leitung, und vorzugsweise in einen arteriellen oder einen venösen Leitungsabschnitt des extrakorporalen Blutkreislaufs oder der Blutkassette hinein, gefördert wird.

Die offenbarte, nicht-erfindungsgemäße Steuer- oder Regelungseinrichtung ist geeignet und vorgesehen und/oder ausgelegt und/oder konfiguriert zur Durchführung des offenbarten, nicht-erfindungsgemäßen Verfahrens.

Die erfindungsgemäße Blutbehandlungsvorrichtung weist einen extrakorporalen Blutkreislauf und/oder eine Blutkassette auf, oder ist hiermit verbunden. Die Blutbehandlungsvorrichtung weist weiter auf oder ist verbunden mit wenigstens einer ersten Quelle für eine Dialysierflüssigkeit oder ein Substituat als einem ersten Fluid; wenigstens einer ersten Leitung, welche sich stromabwärts an die erste Quelle anschließt; wenigstens einer zweiten Quelle für einen Wirkstoff, welcher in der zweiten Quelle in Form eines Konzentrats oder einer Lösung als ein zweites Fluid vorliegt, wobei die zweite Quelle eine Quelle für Citratlösung, für Calciumlösung oder für Heparinlösung ist; wenigstens einer zweiten Leitung, welche sich stromabwärts an die zweite Quelle anschließt; wenigstens einer gemeinsamen Leitung, welche mit einem Abschnitt des extrakorporalen Blutkreislaufs und/oder einem Abschnitt der Blutkassette in Fluidverbindung steht, und in welche sowohl die erste Leitung als auch die zweite Leitung derart münden oder übergehen, dass sowohl die erste Quelle als auch die zweite Quelle mit der gemeinsamen Leitung in Fluidverbindung stehen und/oder wobei die gemeinsame Leitung derart angeordnet ist, dass in ihr das erste Fluid aus der ersten Quelle und das zweite Fluid aus der zweiten Quelle zu einem kombinierten Fluid zusammentreffen können; und einer ersten Fördereinrichtung eines Fluids, welche angeordnet ist, um ein innerhalb der gemeinsamen Leitung vorliegendes Fluid zu fördern.

Die erfindungsgemäße Blutbehandlungsvorrichtung ist vorgesehen und ausgestaltet und/oder ausgestattet zur Durchführung des offenbarten, nicht-erfindungsgemäßen Verfahrens.

Die offenbarte, nicht-erfindungsgemäße Steuereinrichtung ist geeignet und vorgesehen und/oder ausgelegt und/oder konfiguriert zur Durchführung des offenbarten, nicht-erfindungsgemäßen Verfahrens im Zusammenwirken mit den hierzu jeweils erforderlichen Einrichtungen, beispielsweise wie hierin beschrieben.

Ein digitales, insbesondere nichtflüchtiges, Speichermedium, insbesondere in Form eines maschinenlesbaren Trägers, insbesondere in Form einer Diskette, CD, EPROM oder DVD, mit elektrisch auslesbaren Steuersignalen kann derart mit einem programmierbaren Computersystem zusammenwirken, dass die maschinellen Schritte eines offenbarten, nicht-erfindungsgemäßen Verfahrens veranlasst werden.

Dabei können alle, einige oder manche der maschinell durchgeführten Schritte des offenbarten, nicht-erfindungsgemäßen Verfahrens veranlasst werden.

Ein Computerprogramm-Produkt weist einen volatilen, flüchtigen oder auf einem maschinenlesbaren Träger gespeicherten Programmcode zur Veranlassung der maschinellen Schritte des offenbarten, nicht-erfindungsgemäßen Verfahrens, wenn das Computerprogramm-Produkt auf einem Rechner abläuft, auf. Unter einem Computerprogramm-Produkt kann beispielsweise ein auf einem Träger gespeichertes Computerprogramm, ein Embedded System als umfassendes System mit einem Computerprogramm (z. B. elektronisches Gerät mit einem Computerprogramm), ein Netzwerk von computerimplementierten Computerprogrammen (z. B. Client/Serversystem, Cloud Computing System, etc.), oder ein Computer, auf dem ein Computerprogramm geladen ist, abläuft, gespeichert ist, ausgeführt oder entwickelt wird, verstanden werden.

Der Begriff "maschinenlesbarer Träger", wie er hierin verwendet wird, bezeichnet einen Träger, der von Software und/oder Hardware interpretierbare Daten oder Informationen enthält. Der Träger kann ein Datenträger, wie eine Diskette, eine CD, DVD, ein USB-Stick, eine Flashcard, eine SD-Karte oder dergleichen sein.

Ein Computerprogramm weist einen Programmcode auf zur Veranlassung der maschinellen Schritte eines offenbarten, nicht-erfindungsgemäßen Verfahrens, wenn das Computerprogramm auf einem Computer abläuft. Unter einem Computerprogramm kann beispielsweise ein physikalisches, vertriebsfähiges Software-Produkt verstanden werden, welches ein Programm aufweist.

Auch für das offenbarte, nicht-erfindungsgemäße Computerprogramm-Produkt und das offenbarte, nicht-erfindungsgemäße Computerprogramm gilt, dass alle, einige oder manche der maschinell durchgeführten Schritte des offenbarten, nicht-erfindungsgemäßen Verfahrens veranlasst werden.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll erfindungsgemäße Ausführungsformen erläutern.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

In einigen erfindungsgemäßen, beispielhaften Ausführungsformen münden nur die erste und die zweite Leitung in die gemeinsame Leitung (oder gehen in diese über), jedoch keine dritte oder weitere Leitung.

In erfindungsgemäßen Ausführungsformen führt die gemeinsame Leitung, jedenfalls bei bestimmungsgemäßem Gebrauch, kein Blut. Sie ist an entsprechender Stelle angeordnet. Dabei kann sie ihrerseits in eine Blut führende, weitere Leitung münden oder in diese übergehen.

In manchen erfindungsgemäßen, beispielhaften Ausführungsformen ist die gemeinsame Leitung und/oder die erste Fördereinrichtung derart angeordnet, dass mittels der Blutpumpe kein Fluid, welches innerhalb der gemeinsamen Leitung vorliegt, gefördert wird oder werden kann.

In erfindungsgemäßen Ausführungsformen bilden lediglich das erste Fluid und das zweite Fluid das kombinierte Fluid, welches in der gemeinsamen Leitung vorliegt. Weitere Fluide sind in diesen Ausführungsformen an der Zusammensetzung des kombinierten Fluids nicht beteiligt. Weitere Quellen als die erste Quelle und die zweite Quelle stehen, insbesondere stromaufwärts der ersten Fördereinrichtung, nicht mit der gemeinsamen Leitung in Fluidverbindung.

In manchen erfindungsgemäßen, beispielhaften Ausführungsformen ist die gemeinsame Leitung nicht mit einer Wasserquelle oder einer Blutquelle verbunden oder wird von einer solchen gespeist.

In bestimmten erfindungsgemäßen, beispielhaften Ausführungsformen liegt die Dialysierflüssigkeit oder das Substituat gebrauchsfertig in der ersten Quelle vor. Insbesondere eine Vermischung mit Wasser ist bei dieser Darreichung nicht erforderlich, um aus dem Inhalt der ersten Quelle eine gebrauchsfertige Dialysierflüssigkeit oder ein gebrauchsfertiges Substituat zu erzeugen.

In manchen erfindungsgemäßen, beispielhaften Ausführungsformen liegen das erste und/oder das zweite Fluid als Flüssigkeit in der ersten bzw. zweiten Quelle vor, nicht aber in Form von Pulver und/oder in trockener Form.

In einigen erfindungsgemäßen, beispielhaften Ausführungsformen sind die erste und/oder die zweite Quelle ein Beutel.

In manchen erfindungsgemäßen, beispielhaften Ausführungsformen erfolgt kein Anmischen einer Dialysierflüssigkeit, eines Substituats oder des kombinierten Fluids, welches in der gemeinsamen Leitung vorliegt, in einem oder mehreren Tanks.

In gewissen erfindungsgemäßen, beispielhaften Ausführungsformen sind stromauf einer Stelle, an welcher die gemeinsame Leitung in einen blutführenden Abschnitt des extrakorporalen Blutkreislaufs oder der Blutkassette mündet oder in diesen übergeht, nur eine oder nur zwei Fördereinrichtungen angeordnet, welche den Inhalt der gemeinsamen Leitung fördern könnten.

In allen erfindungsgemäßen Ausführungsformen weist die Blutbehandlungsvorrichtung wenigstens eine zweite Fördereinrichtung auf, welche angeordnet ist, um das zweite Fluid innerhalb der zweiten Leitung und/oder in die gemeinsame Leitung hinein zu fördern. Alternativ ist die Blutbehandlungsvorrichtung mit der zweiten Fördereinrichtung verbunden. Das offenbarte, nicht-erfindungsgemäße Verfahren umfasst ferner ein Fördern des zweiten Fluids aus der zweiten Quelle mittels der zweiten Fördereinrichtung innerhalb der zweiten Leitung und/oder in die gemeinsame Leitung hinein.

In gewissen beispielhaften Ausführungsformen ist die zweite Fördereinrichtung keine Fördereinrichtung, welche ausschließlich oder teilweise das zweite Fluid mittels Schwerkraft oder Kapillarwirkung fördert.

In manchen offenbarten, nicht-erfindungsgemäßen, beispielhaften Ausführungsformen umfasst das Verfahren ein Fördern des ersten Fluids aus der ersten Quelle in die erste Leitung und ein Fördern des zweiten Fluids aus der zweiten Quelle in die zweite Leitung in einem, insbesondere vom Anwender, vorbestimmten und/oder verstellbaren Volumen- oder Strömungsverhältnis bezogen auf das erste und das zweite Fluid.

In bestimmten offenbarten, nicht-erfindungsgemäßen, beispielhaften Ausführungsformen umfasst das Verfahren ein Überwachen eines mittels der ersten Fördereinrichtung und/oder der zweiten Fördereinrichtung in die gemeinsame Leitung geförderten Volumens, oder Überwachen einer jeweiligen Förderrate der ersten Fördereinrichtung und/oder der zweiten Fördereinrichtung in die gemeinsame Leitung hinein, jeweils mittels einer ersten und/oder zweiten Überwachungsvorrichtung.

In manchen offenbarten, nicht-erfindungsgemäßen, beispielhaften Ausführungsformen umfasst das Verfahren ein Ermitteln, mittels der ersten und/oder der zweiten Überwachungsvorrichtung, eines Weges, um welchen ein Abschnitt der zweiten Fördereinrichtung, beispielsweise ein Stempel, bewegt wird. Das Verfahren umfasst ferner ein Vergleichen, mittels einer Vergleichseinrichtung, des ermittelten Wegs mit Referenzdaten.

In gewissen offenbarten, nicht-erfindungsgemäßen, beispielhaften Ausführungsformen umfasst das Verfahren ein Zählen, mittels eines Tropfenzählers, von Tropfen des zweiten Fluids. Es umfasst ferner ein Vergleichen, mittels einer Vergleichseinrichtung, der ermittelten Anzahl an Tropfen mit Referenzdaten.

Die Referenzdaten können hierin als Datenbereiche zu verstehen sein. Sie können in Tabellen hinterlegt sein, elektronisch gespeichert sein, usw.

Bei Abweichungen der ermittelten Anzahl an Tropfen von den Referenzdaten können Hinweise oder Alarme ergehen. Alternativ oder ergänzend kann ein Mischungsverhältnis zwischen erstem und zweitem Fluid in Abhängigkeit von und als Reaktion auf eine erkannte Abweichung automatisch angepasst werden.

In einigen offenbarten, nicht-erfindungsgemäßen, beispielhaften Ausführungsformen umfasst das Verfahren das Ermitteln einer Veränderung des Gewichts der ersten Quelle mittels wenigstens einer Waage. Anhand der ermittelten Gewichtsveränderungen kann auf die Förderleistung der ersten Fördereinrichtung oder auf ein durch diese gefördertes Volumen geschlossen werden. Dies kann in Form eines Vergleichs, Abgleichs oder einer Überwachung erfolgen. Das Ergebnis hiervon kann zum korrigierenden Eingreifen in die Steuerung der ersten Fördereinrichtung verwendet werden, welche hierbei zu einer Regelung werden kann. Das Ergebnis von Vergleich, Abgleich oder Überwachung kann als Information oder Alarm an den Benutzer ausgegeben werden.

Beim Schließen von der ermittelten Gewichtsveränderungen auf die Förderleistung der ersten Fördereinrichtung kann diese beispielsweise errechnet, einer Tabelle entnommen oder in Näherung ermittelt oder abgeschätzt werden. Dabei können weitere Informationen wie die Kenntnis eines Anteils des aus der zweiten Quelle stammenden zweiten Fluids an der mittels der ersten Fördereinrichtung geförderten Kombination aus erstem und zweitem Fluid berücksichtigt werden.

In allen erfindungsgemäßen Ausführungsformen weist die zweite Quelle - vorzugsweise ausschließlich, im Wesentlichen oder vorwiegend - Citratlösung, Calciumlösung oder Heparinlösung auf. In bestimmten erfindungsgemäßen, beispielhaften Ausführungsformen weist die zweite Quelle keine Elektrolyte oder nur bestimmte Elektrolyte (in beliebiger Kombination) auf. Zu diesen zählen im Sinne der vorliegenden Erfindung Bikarbonat, Chlorid, Kupfer, Glukose, Eisen, Magnesium, Phosphat, Natrium und Zink.

Eine Citratlösung weist in einigen erfindungsgemäßen Ausführungsformen Citrat auf, wie dies in der US 2006/0037910 A1 und insbesondere in deren Absätzen [0040] bis [0042] beschrieben ist.

In bestimmten erfindungsgemäßen, beispielhaften Ausführungsformen ist die Blutbehandlungsvorrichtung eine Hämodialysevorrichtung, Hämofiltrationsvorrichtung oder Hämodiafiltrationsvorrichtung.

In manchen erfindungsgemäßen, beispielhaften Ausführungsformen weist die Blutbehandlungsvorrichtung eine erste Überwachungsvorrichtung zum Überwachen der Pumpfunktion der ersten Fördereinrichtung auf.

In bestimmten erfindungsgemäßen, beispielhaften Ausführungsformen weist die Blutbehandlungsvorrichtung eine zweite Überwachungsvorrichtung zum Überwachen der Pumpfunktion der zweiten Fördereinrichtung auf.

In manchen erfindungsgemäßen, beispielhaften Ausführungsformen ist die zweite Fördereinrichtung eine Spritzenpumpe mit einem Stempel oder weist eine solche auf. Die zweite Überwachungsvorrichtung ist zum Überwachen der Pumpfunktion der zweiten Fördereinrichtung konfiguriert. Hierzu wird der Weg, um den der Stempel der zweiten Fördereinrichtung bewegt wird, ermittelt. Das Ergebnis der Ermittlung wird mittels einer Vergleichseinrichtung mit Referenzdaten verglichen.

In bestimmten erfindungsgemäßen, beispielhaften Ausführungsformen sind die erste und/oder die zweite Überwachungsvorrichtung zum Überwachen der Pumpfunktion der ersten und/oder zweiten Fördereinrichtung konfiguriert. Hierzu überwachen sie die Funktion der jeweiligen Fördereinrichtung durch Zählen von Tropfen des ersten und/oder zweiten Fluids. Durch Vergleich der ermittelten Anzahl an Tropfen mit Referenzdaten mittels Vergleichseinrichtung wird die jeweilige Fördereinrichtung überwacht.

In allen erfindungsgemäßen Ausführungsformen weist die Blutbehandlungsvorrichtung eine Steuer- oder Regelvorrichtung auf. Die Steuer- oder Regelvorrichtung ist programmiert und/oder konfiguriert um das offenbarte, nicht-erfindungsgemäße Verfahren zum Bereitstellen einer Lösung für eine mittels der Blutbehandlungsvorrichtung und zusätzlich einem extrakorporalen Blutkreislauf und/oder einer Blutkassette durchgeführten Blutbehandlung eines Patienten auszuführen.

Der extrakorporale Blutkreislauf ist in bestimmten Ausführungsformen der vorliegenden Erfindung ein Schlauchset. In jedem Fall ist der extrakorporale Blutkreislauf vorgesehen zum extrakorporalen Leiten von Blut eines Patienten, beispielsweise bei der Hämodialyse, der Hämofiltration, der Hämodiafiltration oder dergleichen.

In einigen erfindungsgemäßen Ausführungsformen ist der extrakorporale Blutkreislauf zumindest abschnittsweise als integraler und ggf. unlösbarer Bestand einer Blutkassette ausgestaltet, in anderen nicht. So kann sich ein frei beweglicher Schlauchabschnitt des extrakorporalen Blutkreislaufs in einem einstückig oder integral auf oder in der Funktionseinrichtung, beispielsweise einer Blutkassette, fortsetzen und umgekehrt.

Eine Blutkassette ist in bestimmten Ausführungsformen der vorliegenden Erfindung eine Einrichtung, welche bei einer Blutbehandlung verwendet wird. Beispiele für Blutkassetten schließen Disposables, Einmal-Blutkassetten ein. Beispielhafte Ausführungsformen einer Blutkassette sind insbesondere in der Anmeldung der Anmelderin mit der Veröffentlichungsnummer DE 10 2009 018 664 A1 mit dem Titel *"Externe Funktionseinrichtung, Blutbehandlungsvorrichtung zum Aufnehmen einer erfindungsgemäßen externen Funktionseinrichtung, sowie Verfahren",* die am 23.04.2009 beim Deutschen Patent- und Markenamt eingereicht wurde, und in der Anmeldung der Anmelderin mit der Veröffentlichungsnummer DE 10 2009 024 468 A1 mit demselben Titel, die am 10.06.2009 beim Deutschen Patent- und Markenamt eingereicht wurde, offenbart.

Der arterielle Leitungsabschnitt des extrakorporalen Blutkreislaufs ist in bestimmten Ausführungsformen der vorliegenden Erfindung jener Leitungsabschnitt, in welchen das den Patientenkörper zum Zwecke der extrakorporalen Blutbehandlung verlassende Patientenblut einströmt und in welchem es sich vor Eintritt in die Blutbehandlungseinrichtung, z. B. einen Dialysator, befindet. In bestimmten Ausführungsformen der vorliegenden Erfindung ist oder umfasst der erste Abschnitt des arteriellen Leitungsabschnitts den arteriellen Nadelanschluss zum Patienten, z. B. den arteriellen Nadelanschluss bei einem Double-Needle-Dialyseverfahren.

Der venöse Leitungsabschnitt des extrakorporalen Blutkreislaufs ist in manchen Ausführungsformen der vorliegenden Erfindung jener Leitungsabschnitt, aus welchem das extrakorporal behandelte Patientenblut nach seiner Behandlung in einer Blutbehandlungseinrichtung, beispielsweise einem Dialysator, zum Patientenkörper hin oder in diesen zurückströmt.

In bestimmten Ausführungsformen der vorliegenden Erfindung entspricht die - während der Blutbehandlung übliche - erste Förderrichtung einer Förderrichtung vom arteriellen Zugang (Blutentnahmestelle) vom Patienten zu einer Blutbehandlungseinrichtung, beispielsweise einem Blutfilter oder einem Dialysator, und anschließend durch den venösen Leitungsabschnitt des extrakorporalen Blutkreislaufs zum venösen Zugang (Blutrückgabestelle). Alle Einrichtungen, die in der ersten Förderrichtung bezogen auf einen Referenzpunkt des Strömungsweges liegen, liegen stromab hierzu. Solche Einrichtungen, die bezogen zum Referenzpunkt entgegen der ersten Förderrichtung liegen, liegen stromauf hierzu.

Die Steuereinrichtung ist in einigen erfindungsgemäßen Ausführungsformen als Regeleinrichtung ausgestaltet.

Die erfindungsgemäße Blutbehandlungsvorrichtung ist in manchen erfindungsgemäßen Ausführungsformen als Hämodialysevorrichtung oder Hämofiltrationsvorrichtung oder Hämodiafiltrationsvorrichtung ausgestaltet.

Die erfindungsgemäße Blutbehandlungsvorrichtung weist wenigstens eine Steuereinrichtung auf. Manche oder alle erfindungsgemäßen Ausführungsformen können einen, mehrere oder alle der oben und/oder im Folgenden genannten Vorteile aufweisen.

Ein mittels mancher Ausführungsformen der vorliegenden Erfindung erzielbarer Vorteil besteht darin, dass Beutel als zweite Quelle verwendet werden können, welche beispielsweise Citrat in hoher Konzentration aufweisen, weshalb der jeweils verwendete Beutel vergleichsweise lange hält. Beutelwechselintervalle können entsprechend lang sein, was Zeit und Mühe zum Wechseln des Beutels erspart, einen nur geringen Lagerplatz für derartige Beutel erfordert und wenig Aufwand bei Beschaffung und Entsorgung bedeutet.

Dabei neigt der Inhalt der zweiten Quelle ungeachtet seiner hohen Konzentration dennoch nicht zu ungleichmäßiger Mischung mit den Patientenblut, wie beispielsweise Koagelbildung, denn der Inhalt wird erst nach Verdünnung mittels Dialysisierflüssigkeit oder Substituat, welche in der gemeinsamen Leitung erfolgt, in das Patientenblut eingebracht.

Ein weiterer erzielbarer Vorteil kann darin bestehen, dass das aus der zweiten Quelle (z. B. einem Beutel) bezogene Volumen, welches - wie auch in den Beispielen der hier beigefügten Figuren - üblicherweise nicht gewogen und damit nicht in die Flüssigkeitsbilanzierung eingeht, zu keinem nennenswerten Flüssigkeitsbilanzfehler führt. Denn das in die gemeinsame Leitung abgegebene Volumen an zweitem Fluid kann aufgrund dessen hoher Konzentration, mit welchem es in der zweiten Quelle vorliegt, vergleichsweise klein oder sehr klein sein. Es kann insbesondere im Vergleich mit dem Volumen des ersten Fluids, welches der ersten Quelle entstammt und als Dialysierflüssigkeit oder Substituat - wie auch in den hier angehängten Figuren - hingegen üblicherweise sehr wohl einer Wiegung unterliegt, sehr klein sein. Der überwiegende Anteil des aus erstem und zweitem Fluid kombinierten Fluids, nämlich das erste Fluid, unterliegt damit durchaus der Bilanzierung, was letztere vorteilhafterweise genauer werden lassen kann.

Umfasst das Verfahren das Ermitteln einer Veränderung des Gewichts der ersten Quelle, so kann anhand der ermittelten Gewichtsveränderungen auf die Förderleistung der ersten Fördereinrichtung oder ein mittels dieser gefördertes Volumen geschlossen werden. Dies kann vorteilhaft einem Überwachen der Tätigkeit der ersten Fördereinrichtung dienen. Das Ergebnis der Überwachung können ein Alarmieren des Anwenders, ein Regeln oder Steuern der ersten Fördereinrichtung und/oder ein anderweitiges Berücksichtigen der mittels - oftmals hochpräzisen, jedenfalls gemessen aus der Steuerbarkeit von Pumpen vergleichsweise präzisen - Waage erhaltenen Kenntnis darüber, dass die erste Fördereinrichtung nicht wie eingestellt fördert, sein, jeweils mit den bekannten, hiermit verbundenen Vorteilen.

Im Folgenden wird die vorliegende Erfindung unter Bezugnahme auf die beigefügten Figuren rein exemplarisch beschrieben. In ihnen bezeichnen selbe Bezugszeichen gleiche oder selbe Komponenten. Es gilt:
- **Fig. 1**: zeigt in vereinfachter Darstellung eine erfindungsgemäße Blutbehandlungsvorrichtung mit einem extrakorporalen Blutkreislauf in einer ersten Ausführungsform; und
- **Fig. 2**: zeigt in vereinfachter Darstellung eine erfindungsgemäße Blutbehandlungsvorrichtung mit einem extrakorporalen Blutkreislauf in einer zweiten Ausführungsform;
- **Fig. 3**: zeigt in vereinfachter Darstellung eine erfindungsgemäße Blutbehandlungsvorrichtung mit einem extrakorporalen Blutkreislauf in einer dritten Ausführungsform;
- **Fig. 4**: zeigt in vereinfachter Darstellung eine erfindungsgemäße Blutbehandlungsvorrichtung mit einem extrakorporalen Blutkreislauf in einer vierten Ausführungsform; und
- **Fig. 5**: zeigt in vereinfachter Darstellung eine erfindungsgemäße Blutbehandlungsvorrichtung mit einem extrakorporalen Blutkreislauf in einer fünften Ausführungsform.

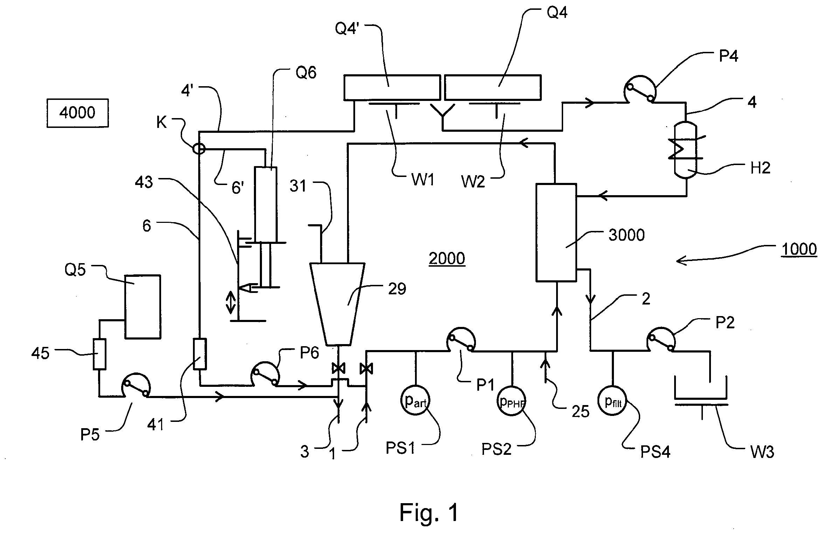

**Fig. 1** zeigt in vereinfachter Darstellung eine erfindungsgemäße Blutbehandlungsvorrichtung 1000 mit einem extrakorporalen Blutkreislauf 2000.

Der extrakorporale Blutkreislauf 2000 weist eine Blutbehandlungseinrichtung 3000, hier exemplarisch einen Blutfilter oder Dialysator, auf oder ist mit dieser verbunden. Ein arterieller Leitungsabschnitt 1 des extrakorporalen Blutkreislaufs 2000 führt Blut vom Gefäßsystem des nicht dargestellten Patienten in Richtung zum Blutfilter 3000. Ein venöser Leitungsabschnitt 3 des extrakorporalen Blutkreislaufs 2000 führt Blut vom Blutfilter 3000 in Richtung zum Gefäßsystem des nicht dargestellten Patienten.

Die in Fig. 1 nur durch einige ihrer Einrichtungen repräsentierte Blutbehandlungsvorrichtung 1000, mittels welcher das hier beschriebene Verfahren abläuft, weist eine Blutpumpe P1 auf. Die Blutpumpe P1 fördert Blut durch Abschnitte des extrakorporalen Blutkreislaufs 2000 und in Richtung zum Blutfilter 3000, wie die kleinen Pfeilspitzen, welche in den Figuren allgemein die Strömungsrichtung angeben, zeigen.

Neben der vorgenannten Pumpe P1 weist die in Fig. 1 gezeigte Anordnung ferner rein optional eine Reihe weiterer Pumpen P2, P4, P5 und P6 auf, die, bis auf die als in der vorliegenden Ausführungsform als zweite Fördereinrichtung zu verstehende Pumpe P6 rein optional vorgesehen sind.

Zu den optionalen Pumpen zählt die Pumpe P4. Sie ist vorgesehen, um Dialysierflüssigkeit aus einer entsprechenden Quelle Q4, beispielsweise einem Beutel, heraus und über eine optional vorhandene Beutelheizung mit Beutel H2 mittels einer Dialysierflüssigkeitsleitung 4 zuzuführen.
Die auf diese Weise zugeführte Dialysierflüssigkeit tritt über eine Dialysatleitung 2, unterstützt durch eine Pumpe P2, wieder aus und kann verworfen werden.

Zu den optionalen Pumpen zählt auch die Pumpe P5, welche eine Kalziumlösung (Ca-Lösung) aus einer Quelle Q5, beispielsweise einem Beutel, heraus- und dem venösen Leitungsabschnitt 3 zuführt.

Nicht optional, bezogen auf die konkreten Ausgestaltung der Fig. 1, ist die Pumpe P6, die im Beispiel der Fig. 1 als erste Fördereinrichtung zu verstehen ist. Pumpe P6 fördert ein kombiniertes Fluid entlang einer Leitung 6, hier als gemeinsame Leitung zu verstehen, in den arteriellen Leitungsabschnitt 1 hinein.

Das kombinierte Fluid entsteht ab einem Kreuzungspunkt K oder einer Verbindung einer ersten Leitung 4' mit einer zweiten Leitung 6' zur gemeinsamen ersten Leitung 6 und ist eine Kombination oder Mischung aus einem ersten Fluid und einem zweiten Fluid.

Das erste Fluid entstammt einer ersten Quelle Q4', hier beispielhaft ein Beutel mit Dialysierflüssigkeit (könnte aber auch Substituat, Kochsalzlösung oder eine andere Lösung oder Flüssigkeit sein), und wird zunächst bis zum Kreuzungspunkt K in der ersten Leitung 4' gefördert.

Das zweite Fluid entstammt einer zweiten Quelle Q6, hier einer Spritze mit Citrat- oder Zitratkonzentrat (Ci-Konzentrat), und wird zunächst bis zum Kreuzungspunkt K in einer zweiten Leitung 6' gefördert. Die Spritze verfügt über eine eigene Fördereinrichtung, eine hier nicht dargestellte Spritzenpumpe. Für den Fachmann ist ersichtlich, dass es einer solchen Spritzenpumpe jedenfalls dann nicht bedarf, wenn die zweite Quelle Q6 keine Spritze sondern beispielsweise ein Beutel ist. In diesem Fall ist die Fördereinrichtung eine Pumpe und nicht optional.

Ab dem Kreuzungspunkt oder Verbindungspunkt K gehen die erste Leitung 4' und die zweite Leitung 6' in die gemeinsame Leitung 6 über oder münden in diese.

Im vorliegenden Beispiel sind die erste Quelle Q4' und die Quelle Q4 voneinander unabhängige Quellen für ein und dasselbe Fluid, nämlich Dialysierflüssigkeit. Von der Erfindung umfasst ist aber auch, dass Q4 und Q4' ein und dieselbe Quelle oder aber miteinander in Fluidverbindung stehende Quellen sind.

Stromauf der Blutpumpe P1 ist ein optionaler arterieller Sensor PS1 vorgesehen, er misst den arteriellen Druck P_art. Stromab der Blutpumpe P1, jedoch stromauf des Blutfilters 3000 und, falls vorgesehen, einer Zugabestelle 25 für Heparin, ist ein weiterer, optionaler Drucksensor PS2 vorgesehen. Er misst den Druck stromauf des Blutfilters 3000 (PHF steht für "prä-Hämofilter").

Ein wiederum weiterer Drucksensor kann als PS4 stromab des Blutfilters 3000, jedoch stromauf der Pumpe P2 in der Dialysatleitung 2 zum Messen eines Filterdrucks des Blutfilters 3000 vorgesehen sein.

Blut, das den Blutfilter 3000 verlässt, durchströmt eine venöse Blutkammer 29, welche eine Entlüftungseinrichtung 31 aufweisen kann.

Im Beispiel der Fig. 1 unterliegen die Quellen Q4 und Q4' sowie das aufgefangene oder verworfene Dialysat optional einer Bilanzierung. Zum Zweck der Bilanzierung sind drei Waagen W1, W2 und W3 vorgesehen.

Die hier exemplarisch gezeigte Bilanzierung entspricht einer gravimetrischem Bilanzierung. Die vorliegende Erfindung umfasst aber auch jede andere Bilanzierung, beispielsweise mittels Bilanzkammern.

Beispielsweise kann der Kreuzungspunkt K mit einer Bilanziereinheit in Fluidverbindung stehen. Jener Volumenanteil am kombinierten Fluid, welcher nicht von der zweiten Quelle Q6 entstammt, also das erste Fluid, kann optional von der Bilanziereinheit ausgeglichen werden.

Die in Fig. 1 gezeigte exemplarische Anordnung weist eine erste Überwachungsvorrichtung 41 auf. Sie ist ausgestaltet zum Überwachen der korrekten Funktion und/oder zum Ermitteln einer Förderleistung oder eines Fördervolumens der ersten Fördereinrichtung, hier der Pumpe P6.

Die in Fig. 1 gezeigte exemplarische Anordnung weist ferner eine zweite Überwachungsvorrichtung 43 auf. Sie ist ausgestaltet zum Überwachen der korrekten Funktion und/oder zum Ermitteln einer Förderleistung oder eines Fördervolumens der zweiten Fördereinrichtung, hier der Spritzenpumpe mit der zweiten Quelle Q6. Die zweite Überwachungsvorrichtung 43 ist, rein optional, erkennbar als Wegesensor ausgestaltet. Sowohl die erste als auch die zweite Überwachungsvorrichtung 41, 43 können als Tropfenzähler ausgestaltet sein. Sowohl die erste als auch die zweite Überwachungsvorrichtung 41, 43 können mit einer in den Figuren nicht dargestellten Vergleichseinrichtung verbunden sein.

Weitere Überwachungsvorrichtungen können, wie in Fig. 1 am Beispiel der Überwachungsvorrichtung 45 gezeigt, ebenfalls vorgesehen sein.

Eine Steuer- oder Regeleinrichtung 4000 ist in Fig. 1 angedeutet. Sie steht in Signalverbindung mit allen relevanten Einrichtungen, jedenfalls oder insbesondere mit der Pumpe P6.

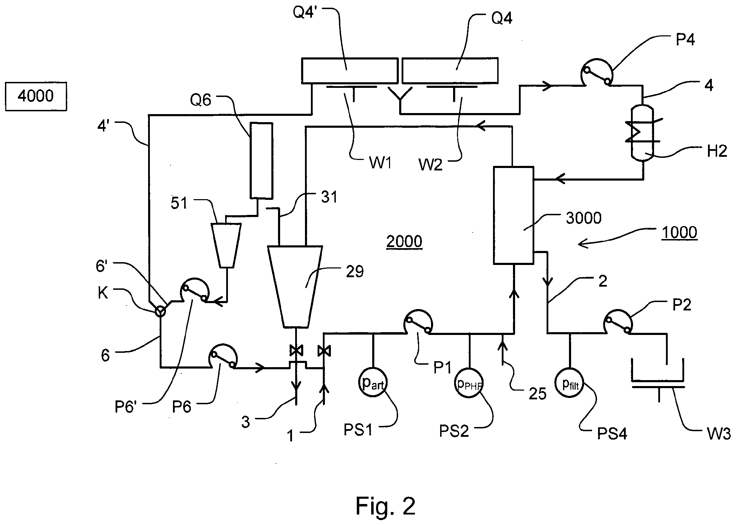

**Fig. 2** zeigt in vereinfachter Darstellung eine erfindungsgemäße Blutbehandlungsvorrichtung mit einem extrakorporalen Blutkreislauf in einer zweiten Ausführungsform.

Pumpe P6' stellt die zweite Fördereinrichtung dar. Pumpe P6' liegt stromab der zweiten Quelle Q6 und stromauf des Kreuzungspunkts K.

Zwischen der zweiten Quelle Q6 und der Pumpe P6' ist eine Tropfkammer 51 vorgesehen. Die Tropfkammer 51 kann der Überwachung mittels einer Überwachungsvorrichtung dienen. Die Ausführungsform der Fig. 2 weist im Bereich der ersten und zweiten Quellen Q4' und Q6 sowie der Leitung 6 rein exemplarisch nur eine Überwachungsvorrichtung auf.

Die zweite Quelle Q6 enthält rein beispielhaft ein beliebiges Konzentrat.

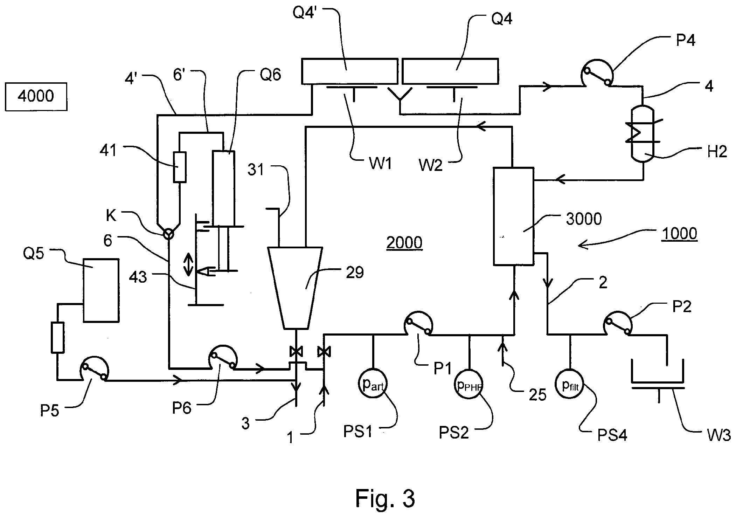

**Fig. 3** zeigt in vereinfachter Darstellung eine erfindungsgemäße Blutbehandlungsvorrichtung mit einem extrakorporalen Blutkreislauf in einer dritten Ausführungsform.

Die Ausführungsform der Fig. 3 unterscheidet sich von jener der Fig. 1 dadurch, dass sich sowohl die erste Überwachungsvorrichtung 41 als auch die zweite Überwachungsvorrichtung 43 in der Leitung 6' befinden. Die erste Leitung 6 weist hingegen rein optional keine Überwachungsvorrichtung mehr auf.

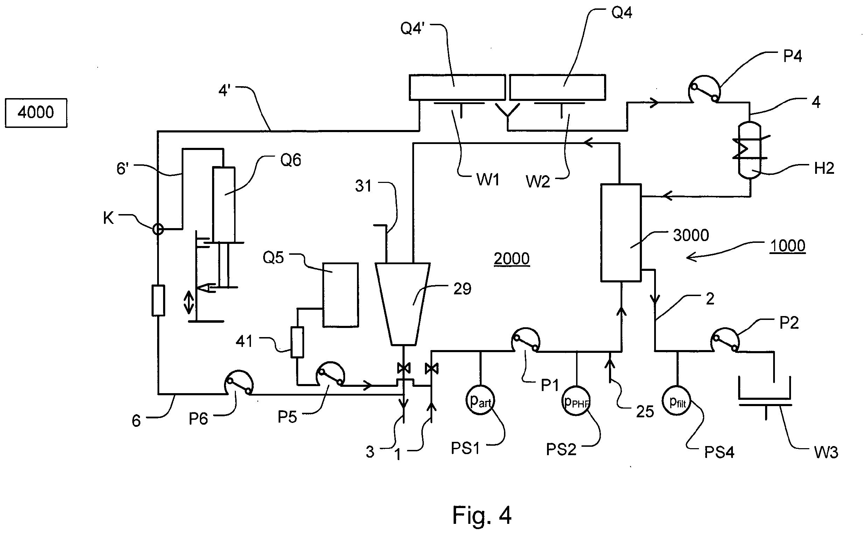

**Fig. 4** zeigt in vereinfachter Darstellung eine erfindungsgemäße Blutbehandlungsvorrichtung mit einem extrakorporalen Blutkreislauf in einer vierten Ausführungsform.

Die Ausführungsform der Fig. 4 unterscheidet sich von jener der Fig. 1 dadurch, dass die Pumpe P6 als erste Fördereinrichtung das kombinierte Fluid nicht in den arteriellen Leitungsabschnitt 1 sondern in den venösen Leitungsabschnitt 3 leitet.

Im Übrigen wird auf die Erläuterungen zur Fig. 1 verwiesen.

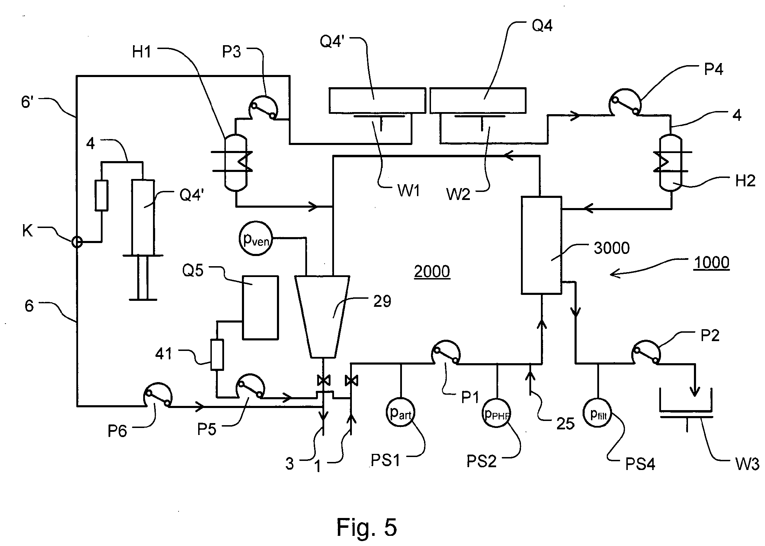

**Fig. 5** zeigt in vereinfachter Darstellung eine erfindungsgemäße Blutbehandlungsvorrichtung mit einem extrakorporalen Blutkreislauf in einer fünften Ausführungsform.

In der Ausführungsform der Fig. 5 weist die erste Quelle Q4' Substituat auf, während die Quelle Q4 Dialysierflüssigkeit enthält. Die Quelle Q4'enthält rein exemplarisch Kalziumkonzentrat.

Im Unterschied zu den Ausführungsformen der vorangegangenen Figuren weist die Anordnung eine weitere Beutelheizung H1 mit einem Beutel für Substituat auf. Zur Förderung des Substituats ist stromab der Quelle Q4', aber stromauf einer Zugabestelle (kann ein Ventil sein oder aufweisen) für das Substituat in Postdilution, eine Pumpe P3 vorgesehen.

Die gemeinsame Leitung 6 mündet in den venösen Leitungsabschnitt 3.

Folgende Merkmale können, obgleich in den Figuren nicht gezeigt, in jeder erfindungsgemäßen Ausführungsform wiederum rein optional und in beliebiger Kombination vorgesehen sein:
Der arterielle Leitungsabschnitt 1 kann eine arterielle Klemme aufweisen.

Der arterielle Leitungsabschnitt 1 kann ein arterielles Septum, optional in Gestalt einer Zugabeeinrichtung, aufweisen.

Der venöse Leitungsabschnitt 3 kann einen venösen Luftblasendetektor/optischen Sensor aufweisen.

Der venöse Leitungsabschnitt 3 kann eine venöse Klemme aufweisen.

Die Blutkassette kann ein Rückschlagventil aufweisen.

Zur Zugabe von Heparin in das Leitungsinnere des extrakorporalen Blutkreislaufs 2000 oder der Blutkassette während einer extrakorporalen Blutbehandlung kann der extrakorporale Blutkreislauf 2000 oder die Blutkassette mittels eines entsprechenden Ports mit einer Zugabestelle für Heparin 25, etwa einer Heparinlösung oder -spritze, verbunden werden oder sein.

Der arterielle Leitungsabschnitt 3 kann einen arteriellen Luftblasendetektor/optischen Sensor aufweisen.

### Bezugszeichenliste

- 1000: Blutbehandlungsvorrichtung
- 2000: extrakorporaler Blutkreislauf
- 3000: Blutbehandlungseinrichtung, Blutfilter oder Dialysator
- 4000: Steuer- oder Regeleinrichtung
- 1: arterieller Leitungsabschnitt
- 2: Dialysatleitung
- 3: venöser Leitungsabschnitt
- 4: Dialysierflüssigkeitsleitung
- 4': erste Leitung, führt aus erster Quelle heraus
- 6: gemeinsame Leitung
- 6': zweite Leitung, führt aus zweiter Quelle heraus
- H1: Beutelheizung mit Beutel
- H2: Beutelheizung mit Beutel

- P1: Blutpumpe
- P2, P3, P4, P5: optionale Pumpen
- P6: Pumpe, erste Fördereinrichtung
- P6': Pumpe, zweite Fördereinrichtung
- PS1: arterieller Sensor, misst den arteriellen Druck P_art
- PS2: optionaler Drucksensor

- PS4: Drucksensor zum Messen eines Filterdrucks

- Q4: Quelle mit Dialysierflüssigkeit
- Q4': erste Quelle
- Q5: Quelle
- Q6: zweite Quelle

- 25: Zugabestelle für Heparin
- 29: venöse Blutkammer
- 31: Entlüftungseinrichtung
- K: Kreuzungspunkt oder Verbindungspunkt der ersten Leitung mit der zweiten Leitung

- W1, W2, W3: Waagen
- 41: erste Überwachungsvorrichtung
- 43: zweite Überwachungsvorrichtung
- 45: Überwachungsvorrichtung
- 51: Tropfkammer

## Patentansprüche

1. Blutbehandlungsvorrichtung (1000), aufweisend einen extrakorporalen Blutkreislauf (2000) und/oder eine Blutkassette, oder hiermit verbunden, wobei die Blutbehandlungsvorrichtung (1000) weiter aufweist oder verbunden ist mit:
- wenigstens eine(r) erste(n) Quelle (Q4') für eine Kochsalzlösung, eine Dialysierflüssigkeit oder ein Substituat als einem ersten Fluid;
- wenigstens eine(r) erste(n) Leitung (4'), welche sich stromabwärts an die erste Quelle (Q4') anschließt;
- wenigstens eine(r) zweite(n) Quelle (Q6) für einen Wirkstoff, welcher in der zweiten Quelle (Q6) in Form eines Konzentrats oder einer Lösung als ein zweites Fluid vorliegt, wobei die zweite Quelle (Q6) eine Quelle für Citratlösung, für Calciumlösung oder für Heparinlösung ist;
- wenigstens eine(r) zweite(n) Leitung (6'), welche sich stromabwärts an die zweite Quelle (Q6) anschließt;
- wenigstens eine(r) gemeinsame(n) Leitung (6), welche mit einem Abschnitt des extrakorporalen Blutkreislaufs (2000) und/oder einem Abschnitt der Blutkassette in Fluidverbindung steht, und in welche sowohl die erste Leitung (4') als auch die zweite Leitung (6') derart münden oder übergehen, dass sowohl die erste Quelle (Q4') als auch die zweite Quelle (Q6) mit der gemeinsamen Leitung (6) in Fluidverbindung stehen;
- einer ersten Fördereinrichtung (P6) eines Fluids, welche angeordnet ist, um das innerhalb der gemeinsamen Leitung (6) vorliegende Fluid zu fördern;
- eine Steuer- oder Regelvorrichtung (4000), welche programmiert und/oder konfiguriert ist, um ein Verfahren zum Bereitstellen einer Lösung für eine mittels der Blutbehandlungsvorrichtung (1000) und zusätzlich einem extrakorporalen Blutkreislauf (2000) und/oder einer Blutkassette durchgeführten Blutbehandlung eines Patienten auszuführen,
wobei die Blutbehandlungsvorrichtung (1000) ferner eine zweite Fördereinrichtung (P6') aufweist, **dadurch gekennzeichnet, dass** die zweite Fördereinrichtung angeordnet ist, um das zweite Fluid innerhalb der zweiten Leitung (6') und/oder in die gemeinsame Leitung (6) hinein zu fördern.

2. Blutbehandlungsvorrichtung (1000) nach Anspruch 1, ferner aufweisend eine erste Überwachungsvorrichtung (41) zum Überwachen der Pumpfunktion der ersten Fördereinrichtung (P6).

3. Blutbehandlungsvorrichtung (1000) nach einem der vorangegangenen Ansprüche, ferner aufweisend eine zweite Überwachungsvorrichtung (43) zum Überwachen der Pumpfunktion der zweiten Fördereinrichtung (P6').

4. Blutbehandlungsvorrichtung (1000) nach einem der vorangegangenen Ansprüche, wobei die zweite Fördereinrichtung (P6') eine Spritzenpumpe mit einem Stempel ist oder aufweist, und wobei die zweite Überwachungsvorrichtung (43) zum Überwachen der Pumpfunktion der zweiten Fördereinrichtung (P6') konfiguriert ist, um die Funktion der zweiten Fördereinrichtung (P6') durch Ermitteln des Weges, um den der Stempel der zweiten Fördereinrichtung bewegt wird, und durch Vergleich des ermittelten Weges mit Referenzdaten mittels Vergleichseinrichtung, zu überwachen.

5. Blutbehandlungsvorrichtung (1000) nach einem der vorangegangenen Ansprüche, wobei die erste und/oder die zweite Überwachungsvorrichtung (41, 43) zum Überwachen der Pumpfunktion der ersten und/oder zweiten Fördereinrichtung (P6, P6') konfiguriert ist, um die Funktion der Fördereinrichtung (P6, P6') durch Zählen von Tropfen des ersten und/oder zweiten Fluids, und durch Vergleich der ermittelten Anzahl an Tropfen mittels Vergleichseinrichtung mit Referenzdaten, zu überwachen.

6. Blutbehandlungsvorrichtung (1000) nach einem der vorangegangenen Ansprüche, ausgestaltet als Hämodialysevorrichtung, Hämofiltrationsvorrichtung oder Hämodiafiltrationsvorrichtung.

## Claims

1. A blood treatment apparatus (1000) comprising an extracorporeal blood circuit (2000) and/or a blood cassette, or connected thereto, the blood treatment apparatus (1000) further comprising or being connected to:
- at least one first source (Q4') for a saline solution, a dialysis liquid or a substitute as a first fluid;
- at least one first line (4') which connects downstream to the first source (Q4');
- at least one second source (Q6) for an active substance which is present in the second source (Q6) in the form of a concentrate or a solution as a second fluid, the second source (Q6) being a source for a citrate solution, a calcium solution or a heparin solution;
- at least one second line (6'), which connects downstream to the second source (Q6');
- at least one common line (6), which is in fluid communication with a portion of the extracorporeal blood circuit (2000) and/or with a portion of the blood cassette, and into which both the first line (4') and the second line (6') lead or merge, such that both the first source (Q4') and the second source (Q6') are in fluid communication with the common line (6),
- a first conveying means (P6) of a fluid, which is arranged to convey fluid present within the common line (6);
- a control or regulating apparatus (4000), which is programmed and/or configured to execute a method for providing a solution for a blood treatment of a patient carried out by means of the blood treatment apparatus (1000) and additionally by means of an extracorporeal blood circuit (2000) and/or a blood cassette,
wherein the blood treatment apparatus (1000) further comprises a second conveying means (P6'),
**characterized in that** the second conveying means is arranged to convey the second fluid within the second line (6') and/or into the common line (6).

2. The blood treatment apparatus (1000) according to claim 1 further comprising a first monitoring apparatus (41) for monitoring the pumping function of the first conveying means (P6).

3. The blood treatment apparatus (1000) according to anyone of the preceding claims further comprising a second monitoring apparatus (43) for monitoring the pumping function of the second conveying means (P6').

4. The blood treatment apparatus (1000) according to anyone of the preceding claims, wherein the second conveying means (P6') is or comprises a syringe pump with a plunger, and wherein the second monitoring apparatus (43) for monitoring the pumping function of the second conveying means (P6') is configured to monitor the function of the second conveying means (P6') by determining the movement path of the plunger of the second conveying means and by comparing the determined path with reference data using a comparison device.

5. The blood treatment apparatus (1000) according to anyone of the preceding claims, wherein the first and/or the second monitoring apparatus (es) (41, 43) for monitoring the pumping function of the first and/or the second conveying means (P6, P6'), is/are configured to monitor the function of the conveying means (P6, P6') by counting drops of the first and/or of the second fluid (s), and by comparing the determined number of drops with reference data using a comparison device.

6. The blood treatment apparatus (1000) according to anyone of the preceding claims, designed as a hemodialysis apparatus, a hemofiltration apparatus or a hemodiafiltration apparatus.

## Revendications

1. Un appareil de traitement du sang (1000) comprenant un circuit sanguin extracorporel (2000) et/ou une cassette à sang, ou relié à celui/celle-ci, l'appareil de traitement du sang (1000) comprenant en outre ou étant relié à:
- au moins une première source (Q4') pour une solution saline, un liquide de dialyse ou un substitut en tant que premier fluide;
- au moins un premier conduit (4'), qui se connecte en aval de la première source (Q4');
- au moins une seconde source (Q6) pour un principe actif présent dans la seconde source (Q6) sous la forme d'un concentré ou d'une solution en tant que second fluide, la seconde source (Q6) étant une source pour une solution de citrate, une solution de calcium ou une solution d'héparine;
- au moins un second conduit (6'), qui se connecte en aval de la seconde source (Q6');
- au moins un conduit commun (6), qui est en communication fluidique avec une partie du circuit sanguin extracorporel (2000) et/ou une partie de la cassette à sang, et dans lequel à la fois le premier conduit (4') et le second conduit (6') débouchent ou mergent de telle sorte que la première (Q4') et la seconde source (Q6') sont en communication fluidique avec le conduit commun (6),
- un premier moyen de transport (P6) de fluide agencé de façon à transporter le fluide présent dans le conduit commun (6) ;
- un appareil de commande ou de régulation (4000) programmé et/ou configuré pour exécuter un procédé permettant de fournir une solution pour un traitement sanguin d'un patient effectué au moyen de l'appareil de traitement du sang (1000) et aussi au moyen d'un circuit sanguin extracorporel (2000) et/ou d'une cassette à sang,
où l'appareil de traitement du sang (1000) comprend en outre un second dispositif de transport (P6'),
**caractérisé en ce que** le second dispositif de transport est agencé de façon à transporter le second fluide dans le second conduit (6') et/ou dans le conduit commun (6).

2. L'appareil de traitement du sang (1000) selon la première revendication comprenant en outre un premier appareil de surveillance (41) pour surveiller la fonction de pompage du premier dispositif de transport (P6).

3. L'appareil de traitement du sang (1000) selon l'une quelconque des revendications précédentes comprenant en outre un second appareil de surveillance (43) pour surveiller la fonction de pompage du second dispositif de transport (P6').

4. L'appareil de traitement du sang (1000) selon l'une quelconque des revendications précédentes, où le second dispositif de transport (P6') est ou comprend une pompe à seringue munie d'un piston, et où le second appareil de surveillance (43) pour surveiller la fonction de pompage du second dispositif de transport (P6'), est configuré de façon à surveiller la fonction du second dispositif de transport (P6') au moyen de la détermination de la trajectoire de déplacement du piston du second dispositif de transport et en comparant la trajectoire déterminée avec des données de référence au moyen d'un dispositif de comparaison.

5. L'appareil de traitement du sang (1000) selon l'une quelconque des revendications précédentes, où le premier et/ou le second appareil(s) de surveillance (41, 43) pour surveiller la fonction de pompage du premier et/ou du second dispositif(s) de transport (P6, P6'), est/sont configuré(s) de façon à surveiller la fonction des dispositifs de transport (P6, P6') par comptage des gouttes du premier et/ou du second fluide(s) et en comparant le nombre de gouttes déterminé avec des données de référence au moyen d'un dispositif de comparaison.

6. L'appareil de traitement du sang (1000) selon l'une quelconque des revendications précédentes, conçu sous la forme d'un appareil d'hémodialyse, d'un appareil d'hémofiltration ou d'un appareil d'hémodiafiltration.
